# EUROPEAN PATENT APPLICATION

(11) **EP 4 596 692 A1**
(43) Date of publication of application: **06.08.2025**
(21) Application number: 23890652.3
(22) Date of filing: 07.11.2023
(51) Int. Cl.: C12N 15/10, C12Q 1/6806, C40B 50/06

(54) **METHOD FOR PROCESSING MIXTURE OF DNA AND RNA**

(30) Priority: 14.11.2022 CN 202211421219; 01.12.2022 CN 202211526565
(71) Applicant: Nanjing Vazyme Biotech Co., Ltd., Nanjing, Jiangsu 210046 (CN)
(72) Inventor: CAO, Lin, Nanjing, Jiangsu 210046 (CN); NIE, Junwei, Nanjing, Jiangsu 210046 (CN); QU, Zhipeng, Nanjing, Jiangsu 210046 (CN); ZHANG, Lijun, Nanjing, Jiangsu 210046 (CN); JIANG, Mingyang, Nanjing, Jiangsu 210046 (CN); ZHENG, Xiaomin, Nanjing, Jiangsu 210046 (CN); AN, Yingjie, Nanjing, Jiangsu 210046 (CN)
(74) Representative: Buzzi, Notaro & Antonielli d'Oulx S.p.A.
(86) International application number: PCT/CN2023/130063
(87) International publication number: WO 2024/104216

(57) **Abstract**

A method for processing a mixture of DNA and RNA. The method involves digesting in advance a portion of the DNA in a sample mixture of DNA and RNA, thereby effectively improving the detection rate of RNA in a combined library of DNA and RNA total nucleic acid samples, helping to detect RNA pathogens and analyze mNGS results.

## Description

### TECHNICAL FIELD

The present application belongs to the field of biotechnology, and specifically relates to a method for processing a mixture of DNA and RNA.

### BACKGROUND

Infectious diseases are one of the common clinical diseases, most of which are local or systemic inflammation or organ dysfunction caused by pathogens such as bacteria, viruses, and fungi, and products thereof, are highly harmful, and lead to a relatively high fatality rate. Infections are one of the leading causes of death in patients with acute and critical illnesses. Severe infections have an acute onset, a rapid progression, and a complex etiology, and therefore it is of vital importance to identify disease-causing pathogenic microorganisms within a short period of time.

The current application of metagenomics Next Generation Sequencing (metagenomics NGS, mNGS) in the detection of pathogenic microbial infections is in full swing, and in particular, mNGS is especially effective in the detection of newly emerging unknown sources of infection, such as the COVID-19 pandemic, the infection source of which was initially confirmed by the mNGS technology as a novel coronavirus. There is a wide variety of pathogenic microorganisms associated with infection, however when classified by the genetic material, they can be divided into RNA and DNA pathogenic microorganisms. Conventional mNGS tests can be used only for microorganisms with one type of nucleic acid each time, such as infection cases caused by bacteria (e.g., *Mycoplasma pneumoniae* and *Mycobacterium tuberculosis*) or RNA viruses (e.g., coronavirus and HIV). However, during the actual diagnosis of infection cases, especially for the diagnosis of intractable and newly emerging cases, it is often difficult to obtain the basic information of pathogenic microorganisms, in other words, it is impossible to confirm whether the pathogenic microorganism is a microorganism derived from DNA or RNA. At this point, if the library construction and then sequencing at the levels of genome (DNA) and transcriptome (RNA) can be performed simultaneously, the identification of the infection source will be certainly speeded up.

The common method of the construction of a DNA library is double-stranded library construction, that is, after DNA is end-blunted and an A tail is added, a sequencing adapter(s) is(are) attached through ligation to complete the library construction. As for RNA, after it is subjected to reverse transcription to form a first strand and a second strand of cDNA, a library is constructed in a manner similar to that of double-stranded DNA, and then sequencing is performed. If there is a need for detection of DNA and RNA simultaneously, it is generally necessary to extract DNA and RNA separately and then construct libraries individually. If the library construction and then detection of DNA and RNA can be performed simultaneously, samples can be saved. After the total nucleic acid is extracted from a single sample, DNA and RNA libraries can be constructed simultaneously, and the genetic information of both can be obtained, so that the purpose of saving samples can be achieved. On the other hand, the genetic information can be obtained more sensitively by library co-construction of the total nucleic acid. For example, in the metagenomics (mNGS) tests, DNA viruses and RNA viruses can be detected simultaneously, which improves the detection efficiency. In tumor detection, if DNA and RNA are detected simultaneously, not only mutations, and insertion/deletion (Indel) mutations can be effectively detected, but also fusion and gene jumping, RNA expression differences, and the likes can be detected at the RNA level, which is of great clinical significance. Therefore, the co-sequencing of DNA and RNA has become the current target for technological breakthroughs.

Library co-construction of DNA and RNA is a relatively cutting-edge technology, and it is rarely reported in the relevant literature. In view of problems such as the great difficulty and high complexity of RNA/DNA co-extraction from pathogenic microorganisms in infection, and large differences in DNA and RNA abundance, it is a big challenge in itself to realize library co-construction of DNA and RNA. At present, in the only existing reports on library co-construction of DNA and RNA, the traditional RNA library construction principle, that is, RNA is subjected to reverse transcription to produce a first strand of cDNA, and then double-stranded cDNA is synthesized by RNase H and DNA polymerase I, is used in the RNA library construction part. Finally, the pre-existing genomic DNA and the double-stranded cDNA generated subsequently in the system can be subjected to conventional DNA library construction schemes, generally fragmentase method and transposase method of library construction. The scheme has some shortcomings, for example, low content and poor stability of RNA viruses in themselves, and serious loss during multiple rounds of experimental operations, making it highly likely that they will be undetected due to below the limit of detection. The efficiency of library construction of trace DNA and RNA is low, such as DNA and RNA samples co-extracted from infectious samples such as sputum, oral swabs, and alveolar lavage fluid.

### SUMMARY

The inventors of the present application have found that after library co-construction of clinical mixed samples of DNA and RNA obtained by co-extraction, in the sequencing results obtained, there is more DNA data and less RNA data, which leads to a low detection rate of RNA, making it very difficult to analyze the results of mNGS in clinical practice. Especially for viral RNA samples with less content in themselves, the low detection rate will cause failure to detect, which will bring great difficulty to clinical judgement. Therefore, it is necessary to improve the RNA detection of co-extracted samples for library co-construction, which is conducive to the mNGS tests.

Since the ratio of DNA to RNA in the real co-extracted nucleic acid samples is not a fixed value, library co-construction and sequencing are performed on the mixed samples containing DNA and RNA at different mass ratios (9 : 1, 5 : 1, and 1 : 1) by simulating the different ratios of DNA to RNA in the extracted samples. If part of DNA in a co-extracted mixture of DNA and RNA is digested first, RNA in the digested mixture of DNA and RNA is subjected to reverse transcription, and the remaining DNA is incorporated before fragmentation, the detection of RNA can be improved, and the higher the ratio of digested DNA, the more RNA is detected, and the sensitivity to low-abundance RNA pathogens is improved.

In a first aspect, the present application provides a method for processing a mixture of DNA and RNA, comprising:
(1) obtaining a mixture of DNA and RNA;
(2) dividing the mixture of DNA and RNA into sample a and sample b in the ratio of N : 1, wherein N is a constant greater than or equal to 2, and the ratio can be volume ratio, mass ratio, or molar ratio, preferably mass ratio;
(3) digesting DNA in sample a to obtain a mixture of DNA and RNA with a reduced DNA content or an RNA mixture substantially free of DNA;
   optionally, purifying or not purifying a product after digesting;
(4) subjecting the mixture of DNA and RNA with a reduced DNA content or the RNA mixture substantially free of DNA to reverse transcription to synthesize a first strand of cDNA;
   optionally, synthesizing a second strand of cDNA after the first strand of cDNA synthesis; and
(5) mixing the product of step (4) with sample b.

In some embodiments, in the method, a nuclease is used to digest DNA in sample a. In some embodiments, the nuclease is a DNA endonuclease, such as one or more of endonuclease dsDNase, T7 endonuclease, endonuclease Vvn, endonuclease DNase I, or endonuclease DNase II, preferably endonuclease DNase I.

In some embodiments, the nuclease is any enzyme that functions to digest single- or double-stranded DNA, but does not digest RNA.

In some embodiments, N is a constant greater than or equal to 2, such as 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.2, 5.5, 5.8, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 19.5.

In some embodiments, N is a constant greater than 19, such as 20, 21, 22, 23, 24, 25, 26, 27, 28, 29, 30, 31, 32, 33, 34, 35, 36, 37, 38, 39, 40, 41, 42, 45, 46, 47, 48, 49, or 50.

In some embodiments, during the reverse transcription, M-MLV reverse transcriptase or AMV reverse transcriptase is used, preferably M-MLV reverse transcriptase.

In some embodiments, the primer used during the reverse transcription is Oligo(dT) or a random hexamer, preferably a random hexamer.

In some embodiments, the mixture of DNA and RNA is obtained by co-extraction, such as magnetic bead method, column chromatography method, and organic solvent method.

In some embodiments, the mass of DNA in the mixture of DNA and RNA is greater than or equal to that of RNA. In some embodiments, the molar amount of DNA in the mixture of DNA and RNA is greater than or equal to that of RNA.

In some embodiments, the method further comprises purifying the reverse transcription product, preferably by magnetic bead purification.

In a second aspect, the present application provides a library co-construction method of DNA and RNA, comprising:
(1) processing a mixture sample of DNA and RNA using the method of the first aspect to obtain a total nucleic acid sample;
(2) optionally, purifying the obtained total nucleic acid sample;
(3) incubating the total nucleic acid sample with a transposase complex to obtain a fragmented nucleic acid sample;
(4) adding primers with an adapter sequence, and performing PCR amplification to obtain a nucleic acid fragment with the adapter; and
(5) optionally, purifying the amplified product.

In some embodiments, the transposase complex comprises a transposase and a transposase recognition core sequence (ME sequence); preferably, the transposase complex further comprises a tag sequence.

In some embodiments, the transposase is a Tn5 transposase, which is any Tn5 transposase known in the art, or an active mutant thereof.

In some embodiments, the Tn5 transposase is a Tn5 transposase having the activity of breaking an RNA-cDNA hybrid chain.

In some embodiments, the primers comprise a first primer with a first adapter sequence and a second primer with a second adapter sequence.

In some embodiments, the adapter sequence is a sequencing matrix-associated sequence used by current or future sequencing platforms (including but not limited to an ion torrent platform, an illumina platform, and an MGI platform).

In some embodiments, the first adapter sequence is P5 adapter sequence of Illumina platform and the second adapter sequence is P7 adapter sequence of Illumina platform, or vice versa.

In some embodiments, the first adapter sequence is P1 adaptor sequence of ion torrent platform and the second adapter sequence is adaptor A of ion torrent platform, or vice versa.

In some embodiments, the first adapter sequence is an upstream splint sequence for single-end tagged library construction of MGI platform and the second adapter sequence is a downstream splint sequence for single-end tagged library construction of MGI platform, or vice versa. In some embodiments, the first adapter sequence is an upstream splint sequence for double-end tagged library construction of MGI platform and the second adapter sequence is a downstream splint sequence for double-end tagged library construction of MGI platform, or vice versa.

In some embodiments, the primers further comprise an index sequence. In some embodiments, the index sequence has, for example, 6-8 bases, preferably 8 bases.

In some embodiments, the primers further comprise a sequence that is identical or complementary to the Tn5 recognition core sequence (ME sequence). In some embodiments, the primers further comprise a sequence complementary to the tag sequence.

In some embodiments, the first primer is N5 primer and the second primer is N7 primer, or vice versa.

In some embodiments, the incubation temperature in step (3) is 40-70°C, e.g. 45-65°C, e.g. 50-60°C, e.g. 55°C for 1-25 minutes, e.g. 3-20 minutes, e.g. 5-15 minutes, e.g. 10 minutes.

In some embodiments, the method further comprises a step of terminating the fragmentation reaction, for example by inactivating the Tn5 transposase, for example by adding ethylenediamine tetraacetic acid (EDTA).

In some embodiments, magnetic bead method, high salt precipitation method, column centrifugation method, or phenol-chloroform extraction method is used in the purification step, preferably magnetic bead method.

In a third aspect, the present application provides a library co-construction method of DNA and RNA, comprising: (1) processing a mixed sample of DNA and RNA using the method of the first aspect to obtain a total nucleic acid sample; (2) breaking down the obtained total nucleic acid sample to obtain a fragmented nucleic acid sample; (3) subjecting the fragmented nucleic acid to end repairing to obtain a nucleic acid fragment; (4) optionally, purifying the nucleic acid fragment; (5) performing adapter ligation; and (6) performing library amplification.

In some embodiments, the breaking down includes, but is not limited to, fragmenting the nucleic acid sample using fragmentase method, chemical method and/or mechanical method. In some embodiments, the mechanical method can be, for example, ultrasonic disruption method, centrifugation method, or heat shock method, and the chemical method is to treat the nucleic acid sample using a chemical reagent to disrupt it into a plurality of fragments.

In some embodiments, the fragmentase method is to use one or more of Endonuclease V, Fragmentase, and T7 endonuclease I.

In some embodiments, the end repairing comprises subjecting the fragmented nucleic acid to end-blunting to enable the ligation of the fragmented nucleic acid with a blunt-ended adapter.

In some embodiments, the end repairing further comprises a step of adding an A, i.e., adding an adenine deoxyribonucleotide at the 3' end after end-blunting, so as to achieve the ligation of the nucleic acid fragment with the adapter via A-T.

In some embodiments, the enzyme by which the fragmented nucleic acid is subjected to end-blunting is, for example, T4 DNA polymerase, and the enzyme used in the step of adding an A can be, for example, Klenow DNA polymerase (3'-5' exo-).

In some embodiments, the adapter is a Y-shaped adaptor or a bubble adapter. In some embodiments, the adapter is a sequencing long adapter or short adapter of illumina platform, or a bubble adapter of MGI platform, or a sequencing adapter of Ion Torrent platform.

In some embodiments, magnetic bead method, high salt precipitation method, column centrifugation method, or phenol-chloroform extraction method is used for purification in the purification step, preferably magnetic bead method.

In a fourth aspect, the present application provides a sequencing library constructed according to the method of the second aspect or the third aspect.

In some embodiments, the number of reads of RNA in the sequencing library is greater than that of reads of RNA in a co-constructed library of an untreated mixed sample of DNA and RNA.

In a fifth aspect, the present application provides a method for detecting a pathogenic microorganism based on mNGS, comprising: (1) obtaining a biological sample; (2) co-extracting nucleic acid to obtain a mixture sample of DNA and RNA; (3) processing the mixture sample of DNA and RNA using the method of the first aspect to obtain a total nucleic acid sample; (4) subjecting the total nucleic acid sample to library construction; and (5) performing sequencing on a sequencer.

In some embodiments, the biological sample is a nasopharyngeal swab, a buccal swab, sputum, alveolar lavage fluid, pleuroperitoneal fluid, cerebrospinal fluid, blood, urine, or stool.

In some embodiments, the mixture sample of DNA and RNA is derived from a host and a pathogenic microorganism, wherein the host can be, for example, a human, and the pathogenic microorganisms can be, for example, one or more of a fungus, a bacterium, a virus, a spirochete, a mycoplasma, a rickettsia, a chlamydia, a prion, and a parasite.

In some embodiments, the pathogenic bacteria can be, for example, *Staphylococcus aureus, Streptococcus pyogenes, Klebsiella pneumoniae, Clostridium difficile, Escherichia coli, Streptococcus pneumoniae, Pseudomonas aeruginosa, Staphylococcus epidermidis, Staphylococcus haemolyticus, Acinetobacter baumannii, Stenotrophomonas maltophilia, Legionella pneumophila, Enterococcus faecalis, Mycobacterium abscessus, Hemophilus parainfluenzae,* or *Mycobacterium tuberculosis.*

In some embodiments, the pathogenic fungi can be, for example, *Candida albicans, Candida tropicalis, Candida glabrata, Aspergillus fumigatus, Coccidioides immitis, Pneumocystis jeroveci, Histoplasma capsulatum,* or *Candida parapsilosis.*

In some embodiments, the pathogenic virus can be, for example, human bocavirus, human parvovirus, human polyoma virus, Epstein-Barr virus, cytomegalovirus, rotavirus, herpes simplex virus, adenovirus, hepatitis B virus, Torque teno virus, varicella-zoster virus, molluscum contagiosum virus, respiratory syncytial virus, vesicular stomatitis virus, or dengue fever virus.

In some embodiments, the library construction method includes, but is not limited to, fragmentase method, mechanical method, and transposase method commonly used in the art.

Compared with the prior art, the present application has the following beneficial effects:
The present application can solve the problem of low RNA detection in library co-construction due to the fact that there is more DNA than RNA during co-extraction, and the detection rate of RNA is increased by 40%-191%. Therefore, the sensitivity of RNA detection is significantly improved, and a co-extracted sample can be detected for DNA and RNA simultaneously.

### Explanation of Relevant Terms

Co-extraction refers to extracting DNA and RNA from a sample simultaneously, by which two types of nucleic acids can be extracted from a sample at one time, so that the utilization rate of the sample (especially for precious samples) is improved, the extraction time is shorten, the cost of extraction reagents is reduced, and the extraction time is decreased.

Library co-construction refers to constructing a library by using DNA template and RNA template simultaneously, so as to obtain the information of DNA and RNA from a sample simultaneously.

mNGS refers to metagenomics Next Generation Sequencing, which is a method for the comprehensive analysis of microbial and host genetic materials (DNA and RNA) from a sample of a patient, and applied for the diagnosis of a variety of infectious diseases, microbiological analysis under disease and health conditions, characterization of human host response to infection transmission, and identification of tumor-associated viruses.

### BRIEF DESCRIPTION OF DRAWINGS

FIG. 1 shows the experimental flow charts of the control group and the test group when 90% of DNA was digested.
FIG. 2 shows the library construction yield diagram when 90% of DNA was digested and the incorporation ratio of DNA template to RNA template was 1 : 1.
FIG. 3 shows the number of sequencing reads when 90% of DNA was digested and the incorporation ratio of DNA template to RNA template was 1 : 1.
FIG. 4 shows the library construction yield diagram when 90% of DNA was digested and the incorporation ratio of DNA template to RNA template was 5 : 1.
FIG. 5 shows the number of sequencing reads when 90% of DNA was digested and the incorporation ratio of DNA template to RNA template was 5 : 1.
FIG. 6 shows the library construction yield diagram when 90% of DNA was digested and the incorporation ratio of DNA template to RNA template was 10 : 1.
FIG. 7 shows the number of sequencing reads when 90% of DNA was digested and the incorporation ratio of DNA template to RNA template was 10 : 1.
FIG. 8 shows the experimental flow charts of the control group and the test group when 95% of DNA was digested.
FIG. 9 shows the library construction yield diagram when 95% of DNA was digested and the incorporation ratio of DNA template to RNA template was 10 : 1.
FIG. 10 shows the number of sequencing reads when 95% of DNA was digested and the incorporation ratio of DNA template to RNA template was 10 : 1.
FIG. 11 shows the experimental flow charts of the control group and the test group when 68% of DNA was digested.
FIG. 12 shows the library construction yield diagram when 68% of DNA was digested and the incorporation ratio of DNA template to RNA template was 10 : 1.
FIG. 13 shows the number of sequencing reads when 68% of DNA was digested and the incorporation ratio of DNA template to RNA template was 10 : 1.

### DETAILED DESCRIPTION

The technical solutions of the present application are further described below with reference to the accompanying drawings and through Detailed Description of Embodiments. However, the following Examples are only simple examples of the present application, and do not represent or limit the scope of protection of the present application, which is subject to the Claims.

In the following Examples, unless otherwise specified, the reagents and consumables used are all purchased from conventional reagent manufacturers in the art; and unless otherwise specified, the experimental methods and technical means used are all conventional methods and means in the art.

### Example 1

The incorporation mass ratio of DNA template to RNA template was 1 : 1.
(1) Template preparation: A total of 10 µL of mixed template of DNA and RNA (DNA template: 1 ng of *Arabidopsis thaliana* DNA + 100 pg of PRV; and RNA template: 1 ng of 293gRNA + 100 pg of PRRSV) was prepared. The template was used in the control group.
(2) Partial digestion of the template in the test group (90% of DNA was digested first): 9 µL of the mixed template of DNA and RNA mentioned above was taken, to which 3 µL of Nuclease-free ddH₂O and 3 µL of wiper (Vazyme #TR501) were added, with a total of 15 µL. In a PCR instrument, the mixture was maintained at 42°C for 2 min, 85°C for 30 sec, and hold at 4°C.
(3) The template in the control group: 5 µL of Nuclease-free ddH₂O was added to 10 µL of untreated mixed template of DNA and RNA, with a total of 15 µL.
(4) Reverse transcription: 2 µL of 10 × RT Mix (reverse transcription buffer, Vazyme #TR501), 2 µL of HiScript III Enzyme Mix (reverse transcriptase, Vazyme #TR501), and 1 µL of Random hexamers (random hexamer primer, Vazyme #TR501) were mixed as a reverse transcription reagent. The test group: 5 µL of the reverse transcription reagent was mixed well with 15 µL of the partially digested template mentioned above in the test group. The control group: 5 µL of the reverse transcription reagent was mixed well with 15 µL of the template mentioned above in the control group. In a PCR instrument, the mixture was maintained at 25°C for 5 min; 37°C for 20 min; 85°C for 5 sec; and hold at 4°C.
(5) Fragmentation: The test group: 20 µL of the reverse transcription product was mixed well with 1 µL of untreated sample, then 10 µL of Tagment buffer (fragmentation reaction buffer, Vazyme #TR501) and 5 µL of Tn5 VR100 (Tn5 transposase, Vazyme #TR501) were added and mixed well, and the mixture was reacted in a PCR instrument at 55°C for 10 min. The control group: 20 µL of the reverse transcription product was mixed well with 1 µL of Nuclease-free ddH₂O, then 10 µL of Tagment buffer (Vazyme #TR501) and 5 µL of Tn5 VR100 (Vazyme #TR501) were added and mixed well, and the mixture was reacted in a PCR instrument at 55°C for 10 min.
(6) Reaction termination: TStop Solution (stop solution, Vazyme #TR501) was thawed at room temperature, and mixed well upside-down for later use. After the reaction was completed, 2 µL of TStop Solution was added to the reaction mixture, vortexed and mixed well, and centrifuged briefly to collect the reaction mixture at the bottom of the tube.
(7) PCR reaction: The reaction system was prepared according to the table below, with a total of 100 µL.

| Component | Volume |
|---|---|
| Reaction product from the previous step | 37 µL |
| N5/N7 primer (Vazyme #TD202) | 10 µL |
| VAHTS HiFi Amplification Mix (Vazyme #TR501) | 50 µL |
| TSE (Vazyme #TR501) | 1 µL |
| Nuclease-free ddH₂O | 2 µL |

The prepared reaction system of the test group was placed in a PCR instrument, and the following reactions were performed.

| Temperature | Time | Number of cycles |
|---|---|---|
| 72°C | 3 min | 1 |
| 95°C | 3 min | 1 |
| 98°C | 20 s | 13 |
| 65°C | 15 s | |
| 72°C | 30 s | |
| 72°C | 5 min | 1 |
| 4°C | Hold | 1 |

The prepared reaction system of the control group was placed in a PCR instrument, and the following reactions were performed.

| Temperature | Time | Number of cycles |
|---|---|---|
| 72°C | 3 min | 1 |
| 95°C | 3 min | 1 |
| 98°C | 20 s | 16 |
| 65°C | 15 s | |
| 72°C | 30 s | |
| 72°C | 5 min | 1 |
| 4°C | Hold | 1 |

### (8) Magnetic bead purification

I. VAHTS DNA Clean Beads (Vazyme #N411) were removed 30 min in advance from 2°C-8°C, and left to stand to equilibrate to room temperature.
II. VAHTS DNA Clean Beads were reversed or vortexed until they were fully mixed, 50 µL (1 ×) was pipetted and added to the product from the previous step, and the mixture was gently pipetted up and down 10 times using a pipette to ensure thorough mixing.
III. The mixture was incubated at room temperature for 10 min to allow DNA to bind to magnetic beads.
IV. The sample was placed on a magnetic rack, and the supernatant was carefully removed after the solution was clarified (about 5 min).
V. The sample was held on the magnetic rack, 200 µL of freshly prepared 80% ethanol was added to rinse the magnetic beads, the mixture was incubated at room temperature for 30 sec, and the supernatant was carefully removed.
VI. Step V was repeated once.
VII. The sample was held on the magnetic rack, and the lip was opened to dry the magnetic beads for about 5-10 min at room temperature.
VIII. The sample was removed from the magnetic rack, 22.5 µL of Nuclease-free H₂O was added, and the mixture was vortexed or gently pipetted up and down using a pipette to ensure thorough mixing. The mixture was left to stand at room temperature for 2 min, and then placed on the magnetic rack. After the solution was clarified (about 5 min), 20 µL of supernatant was pipetted carefully into a new Nuclease-free PCR tube.

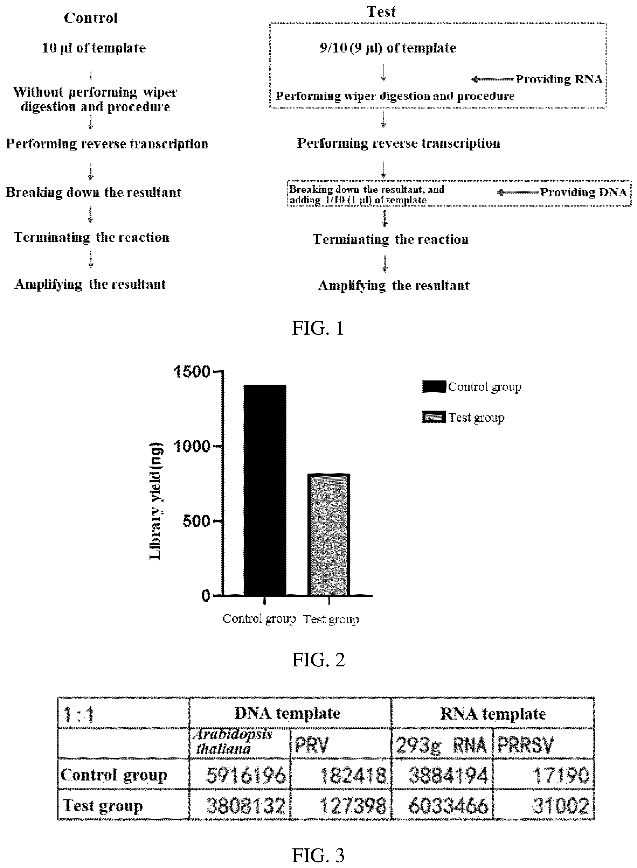

(9) Yield detection: The concentration of the purified products was determined, and the yields are as shown in FIG. 2, wherein the yields of both the test group and the control group were higher than 500 ng, and the products could be sequenced.

(10) Sequencing: The control group and the test group were subjected to second-generation sequencing with a data volume of 20 M, and the detection of different DNA and RNA templates was analyzed.

The detection results are as shown in FIG. 3, wherein the detection sensitivities of the two RNAs in the test group were increased by 55%-81%, as compared to those of the control group, with an increase of 55% ((6033466-3884194)/3884194) for 293gRNA and 81% ((31002-17190)/17190) for PRRSV.

### Example 2

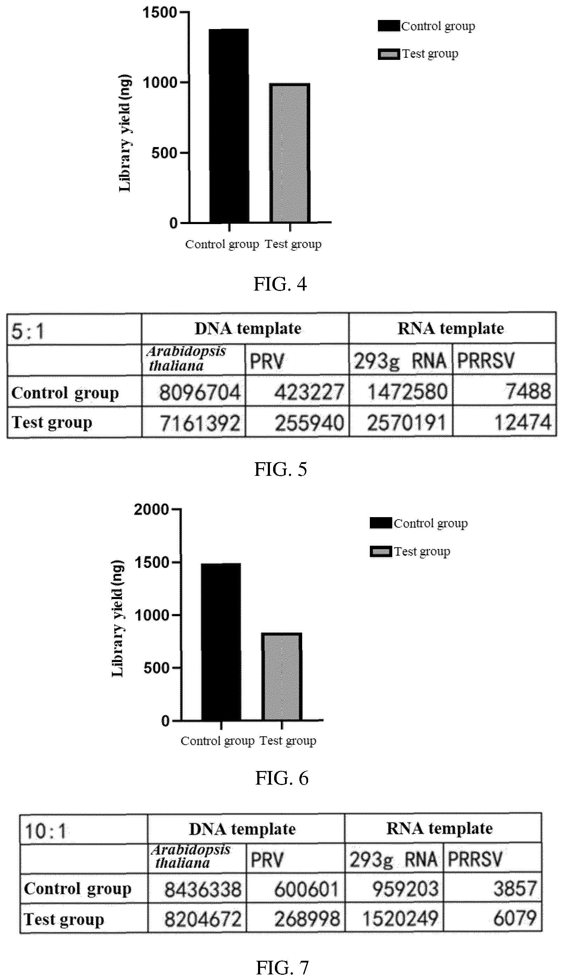

The incorporation mass ratio of DNA template to RNA template was 5 : 1.
(1) Template preparation: A total of 10 µL of mixed template of DNA and RNA (DNA template: 1 ng of *Arabidopsis thaliana* DNA + 100 pg of PRV; and RNA template: 200 pg of 293gRNA + 20 pg of PRRSV) was prepared.
(2) The subsequent experimental steps were the same as those in Example 1 (90% of DNA was digested first).
(3) Yield detection: The concentration of the purified products was determined, and the yields are as shown in FIG. 4, wherein the yields of both the test group and the control group were higher than 500 ng, and the products could be sequenced.
(4) Sequencing: The control group and the test group were subjected to second-generation sequencing with a data volume of 20 M, and the detection of different DNA and RNA templates was analyzed.

The detection results are as shown in FIG. 5, wherein the detection sensitivities of the two RNAs in the test group were increased by 67%-75%, as compared to those of the control group, with an increase of 75% ((2570191-1472580)/1472580) for 293gRNA and 67% ((12474-7488)/7488) for PRRSV.

### Example 3

The incorporation mass ratio of DNA template to RNA template was 10 : 1.
(1) Template preparation: A total of 10 µL of mixed template of DNA and RNA (DNA template: 1 ng of *Arabidopsis thaliana* DNA + 100 pg of PRV; and RNA template: 100 pg of 293gRNA + 10 pg of PRRSV) was prepared.
(2) The subsequent experimental steps were the same as those in Example 1 (90% of DNA was digested first).
(3) Yield detection: The concentration of the purified products was determined, and the yields are as shown in FIG. 6, wherein the yields of both the test group and the control group were higher than 500 ng, and the products could be sequenced.
(4) Sequencing: The control group and the test group were subjected to second-generation sequencing with a data volume of 20 M, and the detection of different DNA and RNA templates was analyzed.

The detection results are as shown in FIG. 7, wherein the detection sensitivities of the two RNAs in the test group were increased by 58%, as compared to those of the control group, with an increase of 58% ((1520249-959203)/959203) for 293gRNA and 58% ((6079-3857)/3857) for PRRSV.

### Example 4

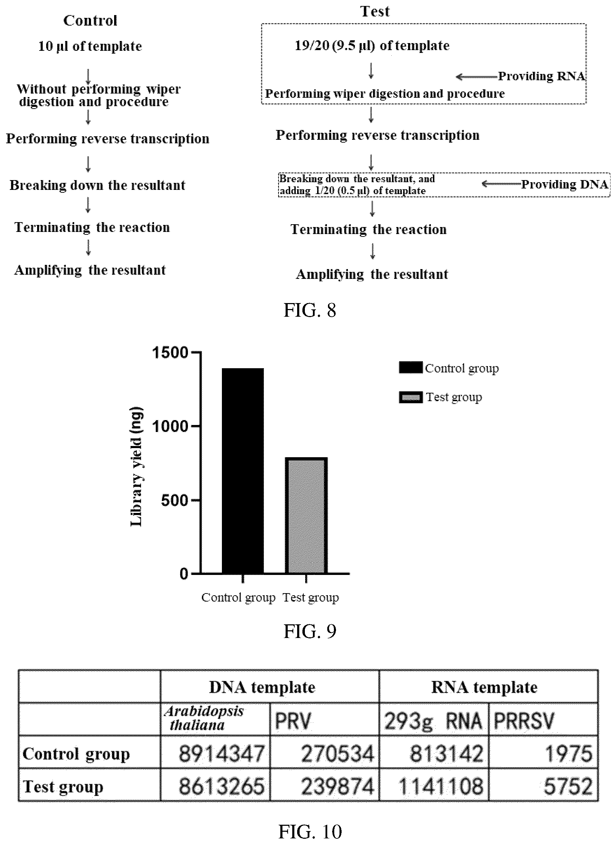

The incorporation mass ratio of DNA template to RNA template was 10 : 1.
(1) Template preparation: A total of 10 µL of mixed template of DNA and RNA (DNA template: 1 ng of *Arabidopsis thaliana* DNA + 100 pg of PRV; and RNA template: 100 pg of 293gRNA + 10 pg of PRRSV) was prepared.
(2) Partial digestion of the template in the test group (95% of DNA was digested first): 9.5 µL of the mixed template of DNA and RNA mentioned above was taken, to which 2.5 µL of Nuclease-free ddH₂O and 3 µL of wiper (Vazyme #TR501) were added, with a total of 15 µL. In a PCR instrument, the mixture was maintained at 42°C for 2 min, 85°C for 30 sec, and hold at 4°C.
(3) The template in the control group: 5 µL of Nuclease-free ddH₂O was added to 10 µL of untreated mixed template of DNA and RNA, with a total of 15 µL.
(4) Reverse transcription: 2 µL of 10 × RT Mix (Vazyme #TR501), 2 µL of HiScript III Enzyme Mix (Vazyme #TR501), and 1 µL of Random hexamers (Vazyme #TR501) were mixed as a reverse transcription reagent. The test group: 5 µL of the reverse transcription reagent was mixed well with 15 µL of the partially digested template mentioned above in the test group. The control group: 5 µL of the reverse transcription reagent was mixed well with 15 µL of the template mentioned above in the control group. In a PCR instrument, the mixture was maintained at 25°C for 5 min; 37°C for 20 min; 85°C for 5 sec; and hold at 4°C.
(5) Fragmentation: The test group: 20 µL of the reverse transcription product was mixed well with 0.5 µL of untreated sample, then 10 µL of Tagment buffer (Vazyme #TR501) and 5 µL of Tn5 VR100 (Vazyme #TR501) were added and mixed well, and the mixture was reacted in a PCR instrument at 55°C for 10 min. The control group: 20 µL of the reverse transcription product was mixed well with 0.5 µL of Nuclease-free ddH₂O, then 10 µL of Tagment buffer (Vazyme #TR501) and 5 µL of Tn5 VR100 (Vazyme #TR501) were added and mixed well, and the mixture was reacted in a PCR instrument at 55°C for 10 min.
(6) The subsequent experimental steps were the same as those in Example 1.
(7) Yield detection: The concentration of the purified products was determined, and the yields are as shown in FIG. 9, wherein the yields of both the test group and the control group were higher than 500 ng, and the products could be sequenced.
(8) Sequencing: The control group and the test group were subjected to second-generation sequencing with a data volume of 20 M, and the detection of different DNA and RNA templates was analyzed.

The detection results are as shown in FIG. 10, wherein the detection sensitivities of the two RNAs in the test group were increased by 40%-191%, as compared to those of the control group, with an increase of 40% ((1141108-813142)/813142) for 293gRNA and 191% ((5752-1975)/1975) for PRRSV.

### Example 5

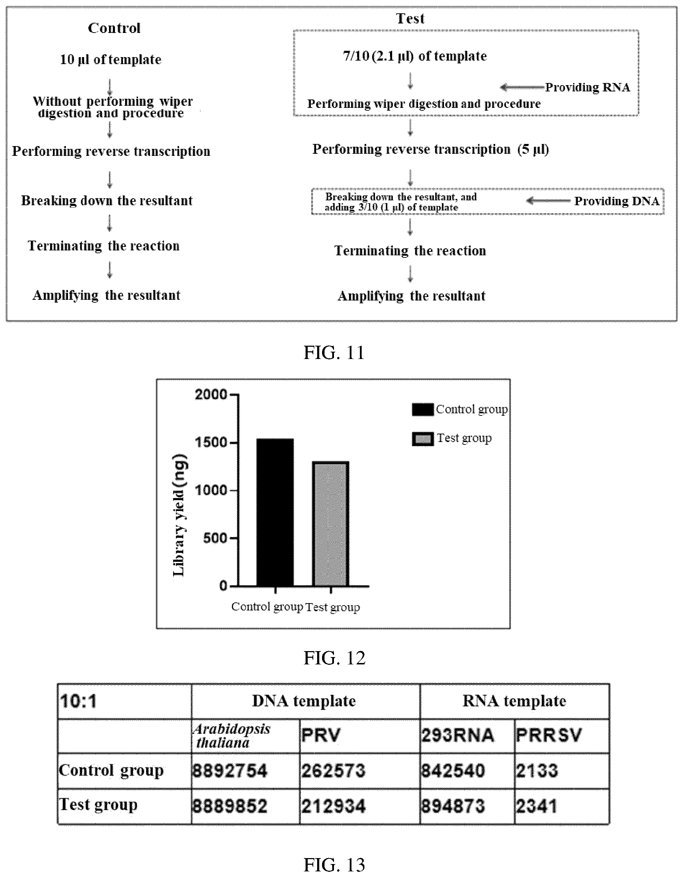

The incorporation mass ratio of DNA template to RNA template was 10 : 1, and the workflows are as shown in FIG. 11.
(1) Template preparation: A total of 3.1 µL of mixed template of DNA and RNA (DNA template: 1 ng of *Arabidopsis thaliana* DNA + 100 pg of PRV; and RNA template: 100 pg of 293gRNA + 10 pg of PRRSV) was prepared.
(2) Partial digestion of the template in the test group (68% of DNA was digested first): 2.1 µL of the mixed template of DNA and RNA mentioned above was taken, to which 9.9 µL of Nuclease-free ddH₂O and 3 µL of wiper (Vazyme #TR501) were added, with a total of 15 µL. In a PCR instrument, the mixture was maintained at 42°C for 2 min, 85 °C for 30 sec, and hold at 4°C.
(3) The template in the control group: 5 µL of Nuclease-free ddH₂O was added to 10 µL of untreated mixed template of DNA and RNA, with a total of 15 µL.
(4) Reverse transcription: 2 µL of 10 × RT Mix (Vazyme #TR501), 2 µL of HiScript III Enzyme Mix (Vazyme #TR501), and 1 µL of Random hexamers (Vazyme #TR501) were mixed as a reverse transcription reagent. The test group: 5 µL of the reverse transcription reagent was mixed well with 15 µL of the partially digested template mentioned above in the test group. The control group: 5 µL of the reverse transcription reagent was mixed well with 15 µL of the template mentioned above in the control group. In a PCR instrument, the mixture was maintained at 25°C for 5 min; 37°C for 20 min; 85°C for 5 sec; and hold at 4°C.
(5) The subsequent experimental steps were the same as those in Example 1.
(6) Yield detection: The concentration of the purified products was determined, and the yields are as shown in FIG. 12, wherein the yields of both the test group and the control group were higher than 500 ng, and the products could be sequenced.
(7) Sequencing: The control group and the test group were subjected to second-generation sequencing with a data volume of 20 M, and the detection of different DNA and RNA templates was analyzed.

The detection results are as shown in FIG. 13, wherein the detection sensitivities of the two RNAs in the test group were increased by 6%-10%, as compared to those of the control group, with an increase of 6% ((894837-842540)/842530) for 293gRNA and 10% ((2341-2133)/2133) for PRRSV.

The applicant has stated that although the detailed methods of the present application are described through the examples described above, the present application is not limited to the detailed methods described above, which means that implementation of the present application does not necessarily depend on the detailed methods described above. It should be apparent to those skilled in the art that any improvements made to the present application, equivalent replacements of raw materials of the product of the present application, additions of adjuvant ingredients to the product of the present application, and selections of specific manners, etc., all fall within the scope of protection and the disclosed scope of the present application.

## Claims

1. A method for processing a mixture of DNA and RNA, comprising:
(1) obtaining a mixture of DNA and RNA;
(2) dividing the mixture into sample a and sample b in the ratio of N : 1, wherein N is a constant greater than or equal to 2;
(3) digesting DNA in sample a to obtain a mixture of DNA and RNA with a reduced DNA content or an RNA mixture substantially free of DNA; optionally, purifying or not purifying a product after digesting;
(4) subjecting the mixture of DNA and RNA with a reduced DNA content or the RNA mixture substantially free of DNA to reverse transcription to synthesize a first strand of cDNA; optionally, synthesizing a second strand of cDNA after the first strand of cDNA synthesis; and
(5) mixing the product of step (4) with sample b.

2. The method according to claim 1, wherein in the method, a nuclease is used to digest DNA in sample a.

3. The method according to claim 1, wherein N is less than or equal to 25.

4. The method according to claim 1, wherein a random hexamer primer is used during the reverse transcription.

5. The method according to any one of claims 1-4, wherein a mass of DNA in the mixture of DNA and RNA is greater than or equal to that of RNA, or a molar amount of DNA in the mixture of DNA and RNA is greater than or equal to that of RNA.

6. The method according to claim 1, wherein the mixture of DNA and RNA is obtained by co-extraction.

7. An improved library co-construction method of DNA and RNA, comprising:
(1) processing a mixture sample of DNA and RNA using the method of any one of claims 1-6 to obtain a total nucleic acid sample;
(2) optionally, purifying the obtained total nucleic acid sample;
(3) incubating the total nucleic acid sample with a transposase complex to obtain a fragmented nucleic acid sample;
(4) adding primers with an adapter sequence, and performing PCR amplification to obtain a nucleic acid fragment with the adapter; and
(5) optionally, purifying the amplified product.

8. The method according to claim 7, wherein the transposase complex comprises a transposase and a transposase recognition core sequence, and the transposase is a Tn5 transposase.

9. The method according to claim 8, wherein the transposase complex further comprises a tag sequence.

10. The method according to claim 9, wherein the primers further comprise an index sequence.

11. The method according to claim 9, wherein the primers further comprise a sequence complementary to the tag sequence.

12. The method according to claim 9, wherein the primers comprise a first primer with a first adapter sequence and a second primer with a second adapter sequence.

13. The method according to claim 12, wherein the first adapter sequence is P5 adapter sequence of Illumina platform, and the second adaptker sequence is P7 adapter sequence of Illumina platform.

14. The method according to claim 7, further comprising a step of terminating the fragmentation reaction, preferably by adding ethylenediamine tetraacetic acid.

15. A library co-construction method of DNA and RNA, comprising:
(1) processing a mixed sample of DNA and RNA using the method of any one of claims 1-6 to obtain a total nucleic acid sample;
(2) breaking down the obtained total nucleic acid sample to obtain a fragmented nucleic acid sample;
(3) subjecting the fragmented nucleic acid to end repairing to obtain a nucleic acid fragment;
(4) optionally, purifying the nucleic acid fragment;
(5) performing adapter ligation; and
(6) performing library amplification.

16. The method according to claim 15, wherein the end repairing comprises subjecting the fragmented nucleic acid to end-blunting, and further comprises adding an adenine deoxyribonucleotide at the 3' end of the fragmented nucleic acid.

17. A sequencing library constructed using the method of any one of claims 7-16.

18. A method for detecting a pathogenic microorganism based on mNGS, comprising:
(1) obtaining a biological sample; (2) co-extracting nucleic acid to obtain a mixture sample of DNA and RNA; (3) processing the mixture sample of DNA and RNA using the method of any one of claims 1-6 to obtain a total nucleic acid sample; (4) subjecting the total nucleic acid sample to library construction; and (5) performing sequencing on a sequencer.
